# EUROPEAN PATENT SPECIFICATION

(11) **EP 2 512 388 B1**
(45) Date of publication and mention of the grant of the patent: **22.04.2015**
(21) Application number: 10787178.2
(22) Date of filing: 18.10.2010
(51) Int. Cl.: A61F 5/451, A61G 9/00

(54) **CONTAINER FOR THE COLLECTION OF URINE OVER A PERIOD OF 24 HOURS**
BEHÄLTER ZUR AUFNAHME VON URIN ÜBER EINEN ZEITRAUM VON 24 STUNDEN
CONTENANT POUR COLLECTE D'URINE PENDANT UNE PÉRIODE DE 24 HEURES

(30) Priority: 14.12.2009 IT PD20090373
(43) Date of publication of application: 24.10.2012
(73) Proprietor: Vacutest Kima S.r.l., 35020 Arzergrande, Padova (IT)
(72) Inventor: CHIARIN, Ulisse, I-35020 Arzergrande PADOVA (IT)
(74) Representative: Mitola, Marco
(86) International application number: PCT/IB2010/054703
(87) International publication number: WO 2011/073804

(56) References cited:
- WO-A1-96/29972
- US-A1- 2006 277 670

## Description

### Field of application

The present invention relates to a container for the collection of urine over a period of 24 hours.

### Background of the invention

Traditional containers for the collection of urine over a period of 24 hours generally comprise two mouths provided with caps, of which a first, of a larger size, used to pour the urine into the container, and a second, of a smaller size, used to draw off the urine from the container.

The urine is drawn off through the aforesaid second mouth using, for example a needle sampling device connected to a vial holder. This drawing off method is effective up to a certain level of filling. Once this level has been exceeded, sampling becomes difficult if not impossible. In particular, to draw off the residual quantities of urine, collected at the bottom of the container, the operator is forced to pour the urine from the container directly into the analysis test tubes. This operation, as well as being impractical, inevitably brings with it the risk of accidental spilling of the contents and consequent contamination of the operator.

US patent application No.US2006/0277670 discloses a container for the collection of urine according to preamble of claim 1. In particular, a container for the collection of urine from a patient is fluidically connected through a first conduit to a second container. The latter is in turn fluidically connected through a second conduit to a pump, which allows to draw off the urine from the collection container and then to transfer the urine to the second container.

### Presentation of the invention

Consequently, the purpose of the present invention is to overcome the drawbacks of the prior art described above, by making available a container for the collection of urine over a period of 24 hours, which enables the residual quantities of fluid to be drawn off directly from the bottom of the container practically and quickly without requiring pouring operations.

A further purpose of the present invention is to make available a container for the collection of urine over a period of 24 hours, which is simple and economical to produce.

### Brief description of the drawings

The technical characteristics of the invention, in relation to the above purposes, can be seen clearly from the contents of the following claims and the advantages of the same will be more clearly comprehensible from the detailed description below, made with reference to the attached drawings, showing one or more embodiments by way of non-limiting examples, wherein:

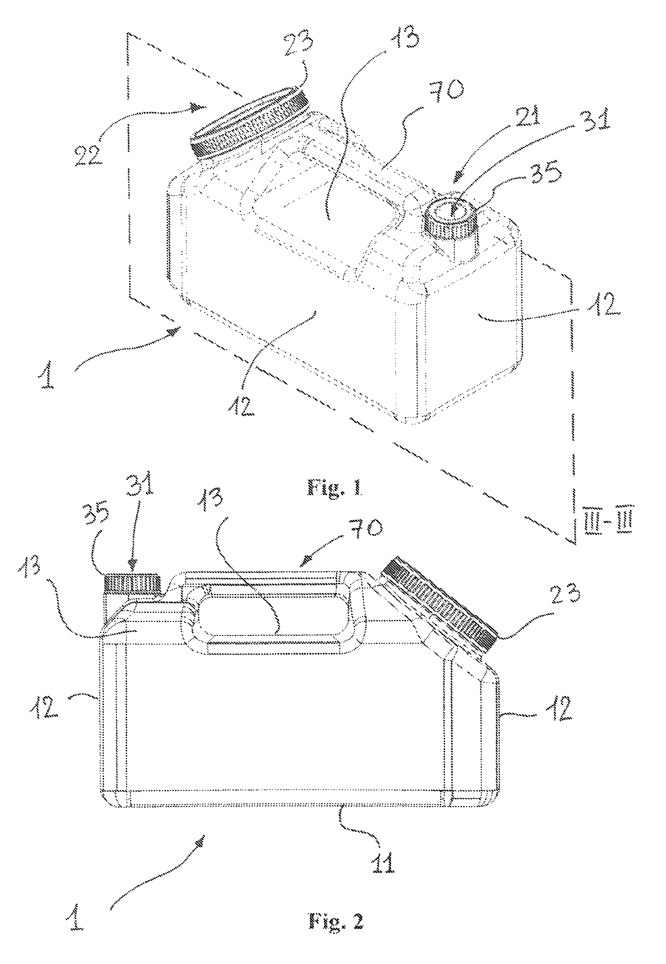

- Figure 1 shows a perspective view of a urine container made according to a first preferred embodiment of the invention;
- Figure 1 shows a lateral view of the urine container illustrated in Figure 1;
- Figure 3 shows a cross section view of the container illustrated in Figure 1, according to the section line III-III therein shown;
- Figure 4 shows the container illustrated in Figure 3 in an operating position suitable for drawing off the residual quantities of urine from the bottom of the container;
- Figure 5 shows an enlarged detail of the container illustrated in Figure 3, relative to the drawing off means of the urine, where in addition a vacuum vial has been shown at the moment of coupling to such drawing off means;
- Figure 6 shows an exploded view of the drawing off means illustrated in Figure 6;
- Figure 7 shows a perspective view of a detail of the drawing off means illustrated in Figure 6, relative to a cup body;
- Figure 8 shows a cross section view of the cup body illustrated in Figure 7, according to the line VIII-VIII shown therein;
- Figure 9 shows a perspective view of a urine container made according to a second preferred embodiment of the invention;
- Figure 10 shows a cross section view of the container illustrated in Figure 9, according to the section line X- X therein shown;
- Figure 11 shows the sectioned container illustrated in Figure 10 in an operating position suitable for drawing off residual quantities of urine from the bottom of the container;
- Figures 12a, 12b and 12c show a container according to the invention associated respectively to a vacuum vial having different suction capacities; and
- Figure 13 shows a detail of the container illustrated in Figure 5, highlighted inside the circle shown therein.

### Detailed description

The container for the collection of urine over a period of 24 hours according to the invention will be globally denoted by reference numeral 1 in the description and attached drawings.

According to the invention, the container 1 comprises a bottom wall 11, a perimetral wall and a top wall 13, which together define the inner volume V of the container 1.

Preferably, the bottom wall 11 is shaped so as to be mainly flat, so as to act as a support base of the container.

The top wall 13 is not necessarily flat in shape, it being able, for example, to define a grip portion or handle 70, as envisaged in the embodiment illustrated in Figure 1.

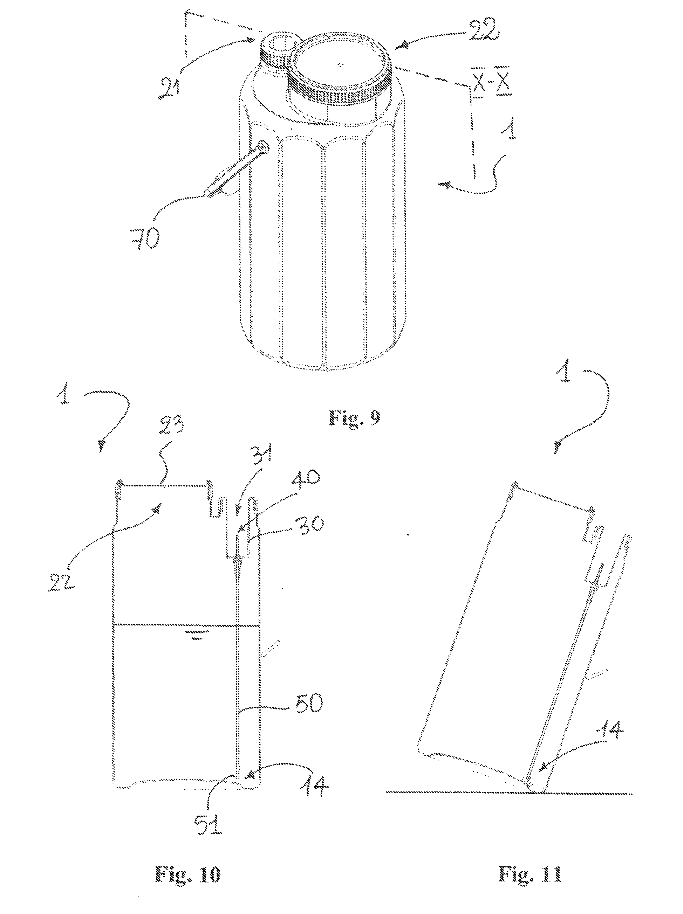

The expression "perimetral wall" is intended to generally comprise both the case of a single, continuous surface (such as, in the case of a perfectly cylindrical container, not shown in the attached drawings) and the case of a discontinuous surface (as shown in the embodiments illustrated in Figures 1 and 9, where the inner volume of the container is delimited by a multiplicity of flat surfaces connected to each other).

Preferably, the container 1 is a substantially parallelepiped shape, as illustrated in Figures 1 and 2, or substantially cylindrical shape, as illustrated in Figure 9. However other three-dimensional shapes may also be envisaged.

Preferably, the container 1 extends mainly in a direction parallel to the bottom wall 11, as envisaged in the embodiment illustrated in Figures 1 and 2 or mainly in an orthogonal direction to the bottom wall 11, as envisaged in the embodiment illustrated in Figure 9. However containers without a main extension direction may also be envisaged.

According to the invention the container 1 comprises at least a first access mouth 21 made on the aforesaid top wall 13 or perimetral wall 12.

According to an essential aspect of the invention, the container 1 comprises means 30, 40, 50 for drawing off the urine collected in the container, firmly associated to said first access mouth 21.

The expression "firmly associated to" is taken to mean that the drawing off means are connected (detachably or not) to the aforesaid access mouth so that no external support or intervention of an operator is required to keep them in position. To such purpose specific connection means of the mouth and the drawing off means may be provided.

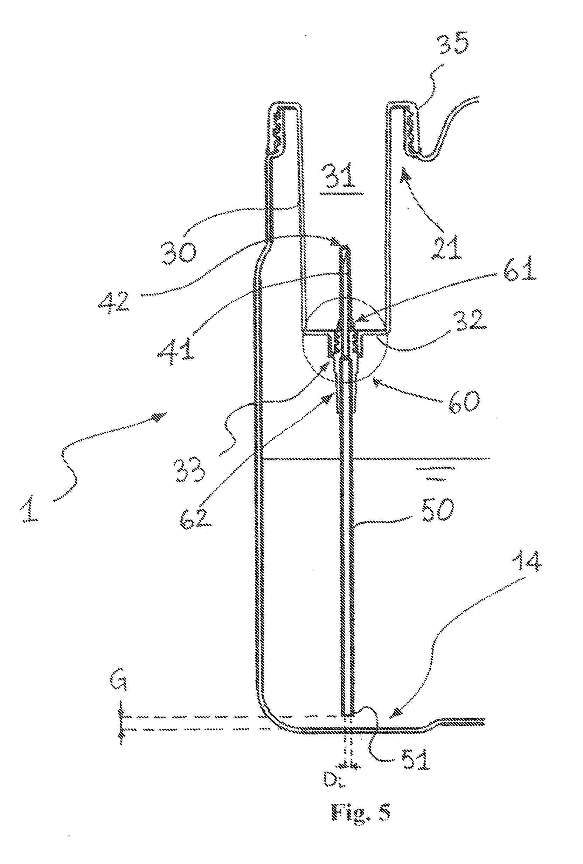

According to a general embodiment, as illustrated for example in Figure 5, such drawing off means 30,40, 50 in turn comprise a cup body 30, inserted - partially or totally - inside the container 1 to close the aforesaid first access mouth 21. Such cup body 30 delimits a chamber 31 for housing a vacuum vial F. On the bottom 32 of such cup body 30 there is an aperture 33 which places the aforesaid chamber 31 in communication with the inner volume V of the container 1.

The drawing off means further comprise means 40 for fluidically coupling the aperture 33 to the vacuum vial F housed in the chamber 31. Such coupling means 40 are associated to the bottom 32 of the cup body, inside the chamber 31, at the aperture 33.

The drawing off means also comprise a tube 50 for sucking the urine collected in the container 1. The tube 50 is fluidically coupled to the bottom aperture 33 and extends from the bottom 32 of the cup body 30 substantially as far as the bottom wall 11 of the container 1.

Operatively, the passage of the urine from the container 1 to the vial F occurs thanks to the difference in pressure between the two environments i.e. container and vial.

As will be explained further below, the tube 50 and fluidically coupling means 40 are appropriately sized in relation to the nominal suction capacity (i.e. vacuum degree) of the vacuum vial F to be used.

Thanks to the fact that the tube 50 extends substantially as far as the bottom wall 11 the drawing off of the urine is possible even when the container is almost empty and only residual quantities of urine are present.

Preferably, as illustrated schematically in Figure 5, between the suction end 51 of the tube 50 and the bottom wall 11 there is a distance G of at least 1 mm, to prevent the suction of any sediments or corpuscles which could obstruct the tube 50 or the coupling means 40.

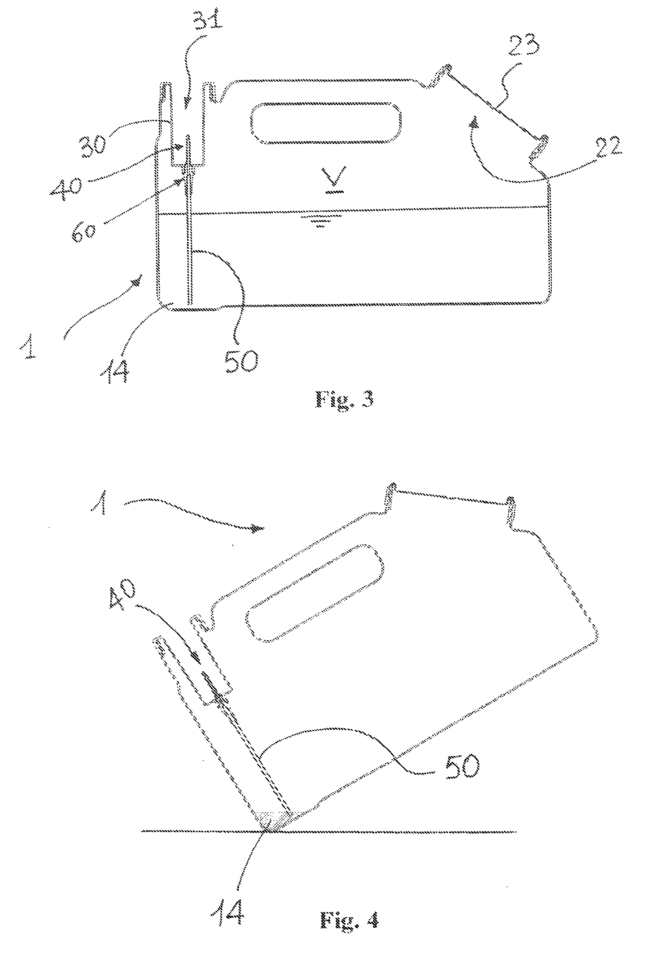

Advantageously, as illustrated for example in Figures 4 and 11, the bottom wall 11 has a depression 14 at the point in which the suction end 51 of the tube 50 is positioned. Such depression 14 facilitates the confluence of the residual urine present in the container 1 towards the suction end 51 and thereby the possibility of drawing off even residual quantities of urine.

In the case in which the depression 14 is envisaged, the aforementioned distance G refers specifically to the distance between the suction end 51 of the tube and the bottom of the depression 14 (as illustrated in Figure 5).

In the embodiment illustrated in Figures 3 and 4, the depression 14 extends only along a shorter side of the rectangular bottom wall.

In the embodiment illustrated in Figures 10 and 11, the depression extends along the entire perimeter of the bottom wall.

Advantageously, especially in the case of the embodiment illustrated in Figures 3 and 4, the bottom wall 11 may be made with a sight inclination to allow the residual urine to flow towards the depression, without the need to tip the container 1.

Operatively, when only residual quantities of urine remain in the container 1, it may however prove useful to tip the container 1 resting it on the area where the tube 50 sucks, so as to facilitate sucking by the tube. This may be particularly opportune in the case of the embodiment illustrated in Figures 10 and 11, even when the level of urine has locally dropped below the sucking height of the tube 50 with the container in a stable position, considering in this specific case the perimetral extension of the depression and the quantity of urine still present.

Preferably, as illustrated in Figures 3 and 10, the first access mouth 21 is made on the top wall 13, substantially on the perpendicular of the depression 14.

According to alternative embodiments (not shown in the attached drawings), the first access mouth 21 (to which the aforesaid drawing off means 30, 40, 50 are associated) may be made on the perimetral wall 12.

In such case, in order to make use of the entire useful volume of the container, the mouth 21 needs to be closed water-tight, so as to prevent leakages of urine from the container, when the level of urine rises above the first mouth. In such case the presence of a second access mouth is also required, made on the top wall to permit pouring of urine into the container making use of the entire useful collecting volume.

It is preferred, consequently, including for reasons of simplicity of construction and ease of operation, to make the first access mouth 21 on the top wall 13 of the container 1. In this position, the level of urine may rise above the bottom of the cup body (when the latter is positioned inside the container), but will not go above the first access mouth 21.

According to a preferred embodiment illustrated in detail in figures 5 to 8, the cup body 30 is provided with a perimetral portion 35, which couples with the rim of the first mouth 21 retaining the cup body inserted coaxially in the mouth 21.

Preferably the perimetral portion 35 and the rim of the mouth 21 couple thanks to a threaded part and to a corresponding counter-threaded part, so as to make the cup body removable from the first mouth 21. Other types of coupling may however be envisaged, for example interference couplings.

Advantageously, the coupling of the perimetral portion and the rim of the mouth may be water-tight, envisaging for example the interposition of an O-ring gasket.

However, as already said, in particular in the case in which the first mouth 21 is made on the top wall 13, given that the container 1 can be used in the stable position at all times - or at most slightly inclined - the water-tightness between the perimetral portion and the rim of the mouth is not essential.

As illustrated in particular in Figure 5, the aforesaid perimetral coupling portion 35 is provided in a distal position in relation to the bottom 32 of the cup body 30. This way, the cup body - in conditions of use - is practically completely inserted inside the container 1. This expedient avoids the presence of annoying projecting elements, improving the ergonomics of the container 1.

Alternative solutions may however be envisaged (not shown in the attached Drawings) wherein the perimetral coupling portion is made in an intermediate position of the cup body 30 or even on the bottom, thereby making the cup body project partially or almost totally from the container 1.

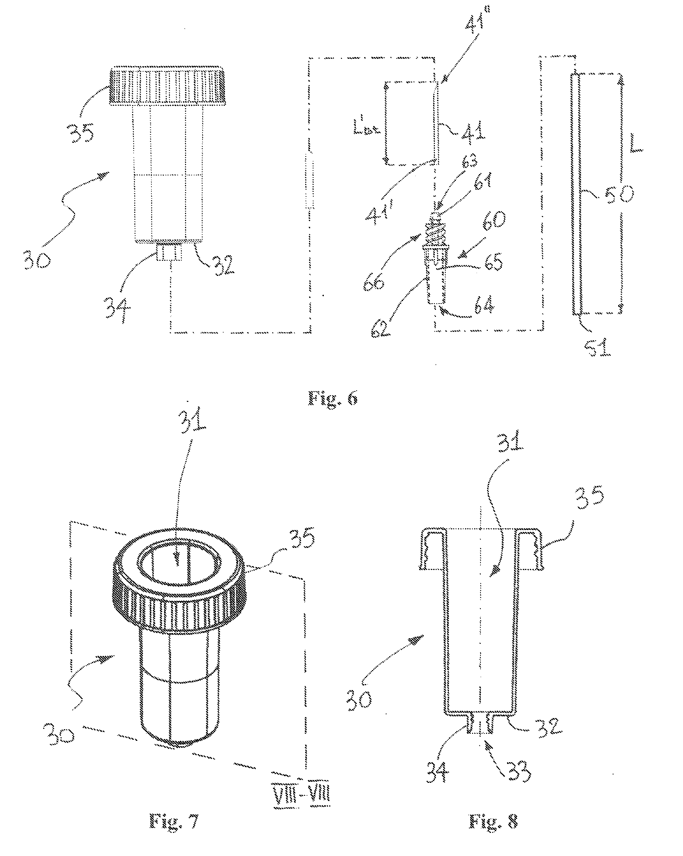

According to a general embodiment, the aforesaid fluidically coupling means 40 comprise a longitudinally hollow needle 41, which projects from the bottom 32 of the cup body inside the chamber 31 housing a vial F, as illustrated for example in Figures 5 and 6.

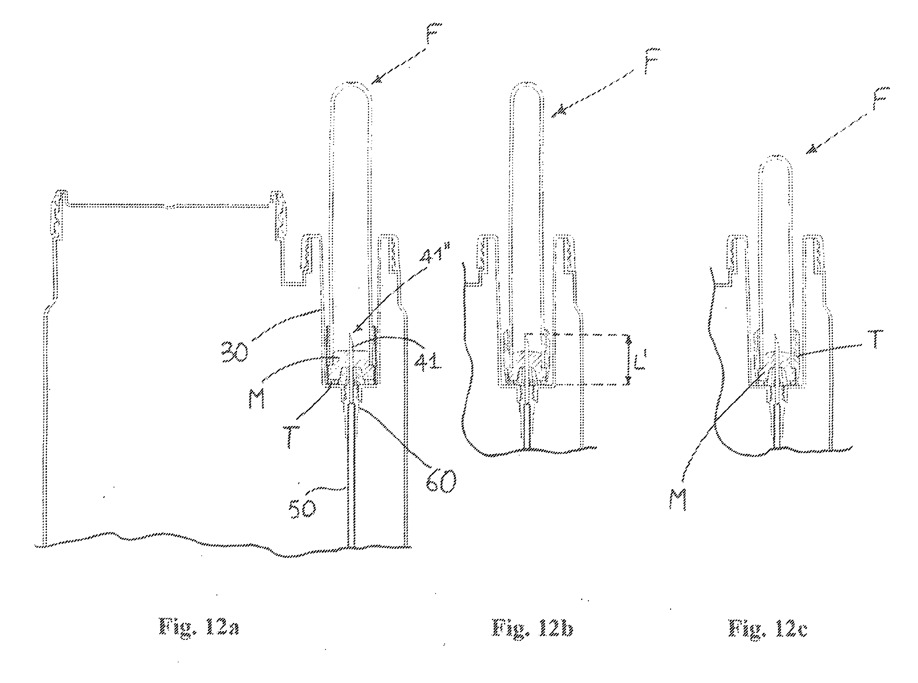

More in detail, the needle 41 is fluidically coupled at its base 41' to the aperture 33 and is provided with a tip 41" able to perforate the membrane M closing a vacuum vial F (see Figures 12a, 12b and 12c), so as to place the inside of the vial with the inner volume V of the container in fluidic communication through the aforesaid aperture 33.

Operatively, as illustrated in Figures 12a, 12b and 12c, a vial F is housed inside the chamber 31 so to bring the cap T against the bottom 32 of the cup body 30, thereby enabling the needle 41 to penetrate inside the vial, perforating the membrane M closing the vial F.

Advantageously, the fluidically coupling means 40 comprises a sheath 42 which protects at least the tip 41" of the needle 41 (preventing the user from accidentally coming into contact with it) and acts as a barrier preventing the leakage of urine (in the case in which the level of urine collected in the container 1 rises above the bottom 32 of the cup body).

Operatively, when a vial F is brought against the bottom 32 of the cup body 30, the tip 41" of the needle is pushed to perforate first the sheath 42 and subsequently the membrane M of the vial. When the vial F is extracted, the sheath 42 - thanks to its elastic properties - returns to its original position, closing the hole made by the tip of the needle. Advantageously, to such purpose, the sheath is made with an elastomer material having a good elastic return. In particular polyisoprene, butyl or styrene butadiene rubber (SBR) and its derivatives may be used. Preferably, the thickness of the sheath wall is 0.2mm to 3.5mm.

The water seal between the cup body 30 and the fluidically coupling means 40 will be spoken of further below.

Preferably, the container 1 comprises a hub 60 associated to the cup body 30 at the aperture 33. The hub 60 associates the needle 41 and the tube 50 to the cup body 30, connecting them fluidically to each other.

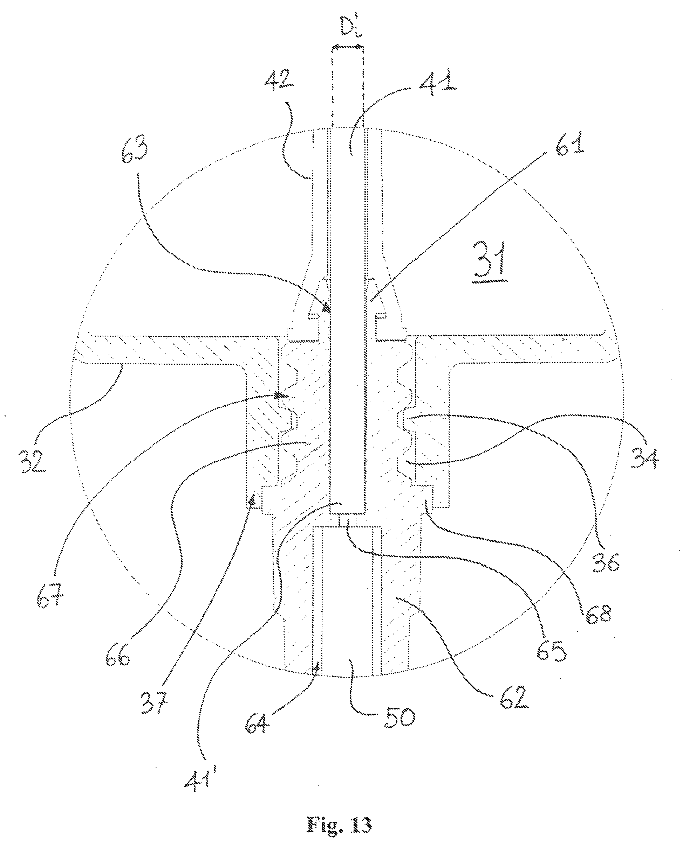

According to the preferred embodiment illustrated in particular in Figures 5 and 13, the hub 60 extends inside the chamber 31 with a first portion 61, on which a first longitudinal seat 63 is made for inserting the base 41' of the needle 41, and inside the container 1 (outside the cup body 30) with a second portion 62, on which a second longitudinal seat 64 is made for inserting a first end 50' of the tube 50. The two inserting seats 63, 64 being fluidically connected to each other by an inner longitudinal channel 65.

Advantageously, the hub 60 is provided with an intermediate attachment portion 66 between the first 61 and second portion 62. By means of such attachment portion 66 the hub 60 is connected to the bottom 32 of the cup body 30 at a fixing seat 34 made therein, which coaxially defines the aperture 33.

Preferably, the attachment portion 66 is defined by a threading 67, a counter threading being envisaged in the aforesaid fixing seat 34. Other types of coupling may however be envisaged, for example interference couplings.

Advantageously, the hub 60 is provided with an annular boss 68 which engages with interference in a counter-shaped annular shoulder 37 made in the aforesaid fixing seat 34 defining the aperture.

According to the embodiment illustrated in Figure 13, the boss 68 is made at the base of said intermediate portion 66, i.e. between the intermediate position 66 and the second portion 62. The counter-shaped annular shoulder 78 is made at the entrance of the fixing seat 34.

Operatively, the hub 60 is inserted inside the fixing seat 34 so as to position the boss 68 against the flat surface of the shoulder 37. The outer cylindrical surface of the annular boss 68 couples with interference with the cylindrical surface of the shoulder 37, thereby creating a water seal without the need to use O-ring gaskets.

Functionally, the aforesaid annular boss 68 of the hub 60 and the corresponding shoulder 37 of the fixing seat 34 create a barrier to the infiltration of urine inside the cup body 30, should the level of urine collected in the container rise above the bottom 32 of the cup body 30.

As already mentioned above, the tube 50 and the fluidically coupling means 40 are appropriately sized in relation to the nominal suction capacity (i.e. vacuum degree) of the vacuum vial F to be used.

Sizing is performed with the objective of reducing pressure drops along the path between the suction end of the tube and the tip of the needle 41.

Advantageously, in the case of containers extending mainly in a direction parallel to the bottom wall 11 (and therefore of reduced height), as envisaged in the embodiment illustrated in Figures 1 and 2, the tube 50 has a length L of 70 to 90mm and an inner diameter Di of 1 to 3mm.

With horizontal containers vacuum vials having a nominal suction capacity of 2 to 6 ml can be used, estimating a maximum suction capacity drop of 25%. Figure 12c shows a container 1 according to the invention coupled to a vacuum vial having a nominal suction capacity of approx. 4 ml (external diameter approx. 13 mm, height approx. 75mm). Vials having a higher suction capacity may also be used.

Advantageously, in the case of containers extending mainly in a direction orthogonal to the bottom wall 11, as envisaged in the embodiment illustrated in Figure 9 (and therefore a greater height than that of the embodiment in Figure 1), the tube 50 has a length L of 140 to 180mm and an inner diameter Di of 1.2 to 3.5 mm.

With vertical containers, vacuum vials having a nominal suction capacity of 6 ml to 12 ml are preferably used, estimating a maximum suction capacity drop of 25%. Figures 12a and 12b show a container 1 according to the invention coupled to a vacuum vial having respectively a nominal suction capacity of approx. 10 ml (external diameter approx. 16 mm, height approx. 100 mm) and 6 ml (external diameter approx. 13 mm, height approx. 100 mm). Vials having a higher or lower suction capacity may also be used, although in the latter case higher suction capacity drops must be estimated.

Advantageously, both with horizontal and vertical containers, a needle 41 having an inner diameter Di' of 0.75 to 1.6 mm is used. Diameters below 0.75 mm lead to excessive pressure drops; for diameters of over 1.6 mm there is a risk that the needle may remove part of the vial membrane and be obstructed by residues of polymer material.

The length of the needle (and of the part projecting inside the housing chamber) must be at least sufficient to allow complete perforation of the membrane M closing the vial F.

In general vials F provided with a rigid cap T fastening the membrane M can be used (illustrated for example in Figures 12 a-c) or vials in which the sealing membrane is heat bonded or glued to the mouth, without any rigid protective cap. In the latter case the length of the needle may be shorter.

Preferably, needles having a total length L'tot of 7 mm (in the case of vials with a thin membrane, heat-bonded or glued) to 35 mm and a length L' of the part projecting into the housing chamber of 2 mm (in the case of vials with thin membranes) to 30 mm are used.

Advantageously, however needles having a length L'tot and L' outside the ranges specified above may also be envisaged.

According to the two preferred embodiments illustrated respectively in Figures 1 and 9, the container 1 is provided with a second access mouth 22.

More in detail, such second access mouth 22 is provided with a normal cap 23 and is destined essentially for pouring urine into the container.

To such purpose, the second mouth 22 may have a larger diameter than the first mouth 21 to facilitate the user when pouring the urine into the container 1.

The diameter of the first mouth 21 is practically imposed by the size of the vacuum vial. A diameter of the first mouth 21 much bigger than that of the cup body may also be envisaged, providing a perimetral coupling portion of an appropriate diameter.

Advantageously, as already mentioned, the container 1 according to the invention may be provided with a grip or handle portion 70.

As illustrated in Figures 1 and 2, the handle 70 may be made in one piece with the top wall 13. Alternatively, as illustrated in Figure 9, the handle 70 may be applied externally to the container 1.

Preferably, the container 1 has a useful inner volume of at least 2 litres and preferably 2 to 3.5 litres. Volumes of over 3 litres, or under 2 litres such as a volume of 1.5 litres may however also be envisaged.

The present invention enables numerous advantages to be achieved, some of which mentioned above.

The container according to the invention makes it possible to directly draw off residual quantities of urine from the bottom quickly and practically.

In fact, the incorporated drawing off means avoid the need for pouring operations of the residual urine, intrinsically unhygienic and operatively laborious.

Thanks to the fact that the container need never be overturned but on the contrary can be left in its stable position or at most inclined slightly, the closure elements of the mouths (i.e. the perimetral coupling portion 35 and, where envisaged, the cap 23 of the second mouth 22) need not necessarily be water-tight. This is to the benefit of constructional simplicity and economy.

The invention thus conceived thereby achieves the intended objectives.

.Obviously, it may assume, in its practical embodiments, forms and configurations different from those illustrated above while remaining within the present sphere of protection.

Furthermore, all the parts may be replaced with technically equivalent parts and the dimensions, shapes and materials used may be varied as required.

## Claims

1. Container for the collection of urine over a period of 24 hours, the inner volume of which is delimited by a bottom wall (11), a perimetral wall (12) and by a top wall (13), said container comprising at least a first access mouth (21), made on said top wall (13) or on said perimetral wall (12), and means for drawing off (30, 40, 50) the urine collected in the container, **characterised by** the fact that said means for drawing off (30, 40, 50) the urine collected in the container are permanently associated to said first access mouth (21) and comprise:
- a cup body (30) inserted, partially or totally, inside said container (1) to close said first access mouth (21), said cup body (30) delimiting a chamber (31) for the housing of a vacuum vial (F), on the bottom (32) of said cup body (30) there being an aperture (33) which places said chamber (31) in communication with the inner volume (V) of said container (1);
- means (40) for fluidically coupling said aperture (33) to said vial (F) housed in said chamber (31), said coupling means (40) being associated to the bottom (32) of said cup body, inside said chamber (31), at said aperture (33); and
- a tube (50) for sucking the urine collected in said container (1), said tube being fluidically coupled to said aperture (33) and extending from the bottom (32) of said cup body (30) substantially as far as said bottom wall (11) of said container (1).

2. Container according to claim 1, wherein between the suction end (51) of said tube (50) and said end wall (11) there is a distance (G) of at least 1 mm.

3. Container according to claim 1 or 2, wherein said bottom wall (11) has a depression (14) at the point in which the suction end (51) of said tube (50) is positioned, said depression (14) facilitating the confluence of the residual urine present in said container (1) towards said suction end (51).

4. Container according to claim 3, wherein said first access mouth (21) is made on said top wall (13) substantially on the perpendicular of said depression (14).

5. Container according to any of the previous claims, wherein said tube (50) has an inner diameter (Di) of 1 to 3 mm and a length (L) of 70 to 90 mm.

6. Container according to any of the claims from 1 to 4, wherein said tube (50) has an inner diameter (Di) of 1.2 to 3.5 mm and a length (L) of 140 to 180 mm.

7. Container according to any of the previous claims, wherein said cup body (30) is retained coaxially in said first access mouth (21) by means of a perimetral portion (35), which couples with the rim of said first access mouth (21).

8. Container according to any of the previous claims, wherein said fluidically coupling means (40) comprise a longitudinally hollow needle (41) which projects inside said chamber (31) from the bottom (32) of said cup body, said needle (41) being fluidically coupled at its base (41') to said aperture (33) and being provided with a tip (41") to perforate the membrane (M) closing said vial (F) so as to place in fluidic communication the inside of said vial (F) with the inner volume (V) of said container (1) by means of said aperture (33).

9. Container according to claim 8, wherein said fluidically coupling means (40) comprises a sheath (42) which protects at least the tip (41") of said needle (41) and acts as a barrier preventing the leakage of urine from said container through said needle (41).

10. Container according to claim 8 or 9, wherein said longitudinally hollow needle (41) has an inner diameter (Di') of 0.75 mm to 1.6 mm.

11. Container according to claim 8, 9 or 10, wherein said longitudinally hollow needle (41) has a total length (L'tot) of 7 to 35 mm and length of the section projecting inwards (L') of 2 to 30 mm.

12. Container according to one or more of the claims from 8 to 11, comprising a hub (60) associated to said cup body 30 at said aperture (33), said hub (60) associating said needle (41) and said tube (50) to said cup body (30), connecting them fluidically to each other.

13. Container according to claim 12, wherein said hub (60) extends inside said chamber (31) with a first portion (61), on which a first longitudinal seat (63) is made for inserting the base (41') of said needle (41), and inside said container (1) with a second portion (62), on which a second longitudinal seat (64) is made for inserting a first end (50') of said tube (50), said two inserting seats (63, 64) being fluidically connected to each other by an inner longitudinal channel (65).

14. Container according to claim 13, wherein said hub (60) is provided with an intermediate attachment portion (66), between said first (61) and said second portion (62), by means of which it is connected to the bottom (32) of said cup body (30) in correspondence of a fixing seat (34) which is made on the bottom (32) of said cup body (30) and which axially defines said aperture (33).

15. Container according to one or more of the claims from 12 to 14, wherein said hub (60) is provided with an annular boss (68) which engages with interference in a counter-shaped annular shoulder (37) made in said fixing seat (34), thereby creating a barrier to the entrance of urine from said container (1) to said housing chamber (31).

16. Container according to claims 14 and 15, wherein said annular boss (68) is made at the base of said intermediate portion 66 and said counter-shaped annular shoulder (78) is made at the entrance of said fixing seat (34).

17. Container according to any of the previous claims, comprising a second access mouth (22).

18. Container according to any of the previous claims comprising a transport handle (70).

19. Container according to any of the previous claims, having a useful inner volume of 2 to 3.5 litres.

## Patentansprüche

1. Behälter zum Sammeln von Urin über eine Dauer von 24 Stunden, dessen inneres Volumen, welches durch eine Bodenwand (11), eine Perimetralwand (12) und durch eine Oberseitenwand (13) begrenzt ist, der Behälter umfassend zumindest eine erste Zugangsmündung (21), welche an der Oberseitenwand (13) oder an der Perimetralwand (12) gemacht ist, und Mittel zum Ablassen (30, 40, 50) des im Behälter gesammelten Urins, **gekennzeichnet durch** die Gegebenheit, dass die Mittel zum Ablassen (30, 40, 50) des im Behälter gesammelten Urins permanent mit der ersten Zugangsmündung (21) assoziiert sind und umfassen:
- einen Becherkörper (30), welcher teilweise oder vollständig innerhalb des Behälters (1) eingeführt ist, um die erste Zugangsmündung (21) zu schließen, der Becherkörper (30) eine Kammer (31) für die Unterbringung einer Vakuumflasche (F) abgrenzt, wobei sich am Boden (32) des Becherkörpers (30) eine Öffnung (33) befindet, welche die Kammer (31) in Verbindung mit dem inneren Volumen (V) des Behälters (1) setzt;
- Mittel (40) zum fluidtechnischen Koppeln der Öffnung (33) mit der in der Kammer (31) untergebrachten Flasche (F), wobei die Koppelmittel (40) mit dem Boden (32) des Becherkörpers assoziiert sind, innerhalb der Kammer (31) an der Öffnung (33); und
- ein Rohr (50) zum Saugen des im Behälter (1) gesammelten Urins, wobei das Rohr fluidtechnisch an die Öffnung (33) gekoppelt ist und sich vom Boden (32) des Becherkörpers (30) im Wesentlichen so weit wie die Bodenwand (11) des Behälters (1) erstreckt.

2. Behälter nach Anspruch 1, wobei zwischen dem Saugende (51) des Rohrs (50) und der Endwand (11) eine Distanz (G) von zumindest 1 mm ist.

3. Behälter nach Anspruch 1 oder 2, wobei die Bodenwand (11) eine Vertiefung (14) an dem Punkt hat, an welchem das Saugende (51) des Rohrs (50) positioniert ist, wobei die Vertiefung (14) die Konfluenz des im Behälter (1) residualen Urins gegen das Saugende (51) ermöglicht.

4. Behälter nach Anspruch 3, wobei die erste Zugangsmündung (21) an der Oberseitenwand (13) im Wesentlichen auf der Senkrechten der Vertiefung (14) gemacht ist.

5. Behälter nach irgendeinem der vorangegangenen Ansprüche, wobei das Rohr (50) einen inneren Durchmesser (Di) von 1 bis 3 mm und eine Länge (L) von 70 bis 90 mm hat.

6. Behälter nach irgendeinem der Ansprüche von 1 bis 4, wobei das Rohr (50) einen inneren Durchmesser (Di) von 1,2 bis 3,5 mm und eine Länge (L) von 140 bis 180 mm hat.

7. Behälter nach irgendeinem der vorangegangenen Ansprüche, wobei der Becherkörper (30) koaxial in die erste Zugangsmündung (30) mittels eines perimetralen Abschnitts (35) gehalten ist, welcher mit dem Rand der ersten Zugangsmündung (21) koppelt.

8. Behälter nach irgendeinem der vorangegangenen Ansprüche, wobei die fluidtechnischen Koppelmittel (40) eine longitudinale hohle Nadel (41) umfassen, welche in die Kammer (31) vom Boden (32) des Becherkörpers hineinragt, wobei die Nadel (41) fluidtechnisch an ihrer Basis (41') an der Öffnung (33) gekoppelt ist und mit einer Spitze (41 ") ausgestattet ist, um die Membran (M), welche die Flasche (F) schließt, zu perforieren, um das Innere der Flasche (F) mit dem inneren Volumen (V) des Behälters (1) mittels der Öffnung (33) in fluidtechnischer Verbindung zu setzen.

9. Behälter nach Anspruch 8, wobei die fluidtechnischen Kopplungsmittel (40) eine Hülle (42) umfassen, welche zumindest die Spitze (41 ") der Nadel (41) schützt und als eine Barriere fungiert, welche die Leckage von Urin aus dem Behälter durch die Nadel (41) verhindert.

10. Behälter nach Anspruch 8 oder 9, wobei die longitudinale hohle Nadel (41) einen inneren Durchmesser (Di') von 0,75 mm bis 1,6 mm hat.

11. Behälter nach Anspruch 8, 9 oder 10, wobei die longitudinale hohle Nadel (41) eine Gesamtlänge (L'tot) von 7 bis 35 mm und eine Länge des nach innen hineinragenden Abschnitts (L') von 2 bis 30 mm hat.

12. Behälter nach einem oder mehreren der Ansprüche 8 bis 11, umfassend einen Hub (60) (engl. hub) an der Öffnung (33) assoziiert mit dem Becherkörper (30), wobei der Hub (60) die Nadel (41) und das Rohr (50) an den Becherkörper (30) assoziiert und diese fluidtechnisch miteinander verbindet.

13. Behälter nach Anspruch 12, wobei der Hub (60) sich mit einem ersten Abschnitt (61), auf welchem ein longitudinaler Sitz (63) zum Einfügen der Basis (41') der Nadel (41) gemacht ist, in die Kammer (31) erstreckt, und in den Behälter (1) mit einem zweiten Abschnitt (62), auf welchem ein zweiter longitudinaler Sitz (64) gemacht ist um ein erstes Ende (50') vom Rohr (50) einzufügen, wobei die Einfügsitze (63, 64) fluidtechnisch durch einen inneren longitudinalen Kanal (65) miteinander verbunden sind.

14. Behälter nach Anspruch 13, wobei der Hub (60) mit einem dazwischenliegenden Befestigungsabschnitt (66) ausgestattet ist, zwischen dem ersten (61) und dem zweiten Abschnitt (62), mittels welchem es mit dem Boden (32) des Becherkörpers (30) in Korrespondenz mit einem Fixiersitzes (34), welcher auf dem Boden (32) des Becherkörpers (30) gemacht ist und welcher die Öffnung (33) axial definiert, verbunden ist.

15. Behälter nach einem oder mehreren der Ansprüche von 12 bis 14, wobei der Hub (60) mit einem ringförmigen Vorsprung (68) (engl. annular boss) ausgestattet ist, welcher mit Überlagerung in eine in dem Fixiersitz (34) gemachte entgegengeformte ringförmige Schulter (37) eingreift, dadurch schaffend eine Barriere zum Eingang des Urins vom Behälter (1) zur Unterbringungskammer (31).

16. Behälter nach Ansprüche 14 und 15, wobei der ringförmige Vorsprung (68) an der Basis des dazwischenliegenden Abschnitt (66) gemacht ist und die entgegengeformte ringförmigen Schulter (78) am Eingang des Fixiersitzes (34) gemacht ist.

17. Behälter nach irgendeinem der vorangegangenen Ansprüche, umfassend eine zweite Zugangsmündung (22).

18. Behälter nach irgendeinem der vorangegangenen Ansprüche umfassend einen Transportgriff (70).

19. Behälter nach einem der vorangegangenen Ansprüche, mit einem brauchbaren inneren Volumen von 2 bis 3,5 Liter.

## Revendications

1. Contenant pour collecte d'urine pendant une période de 24 heures, dont le volume intérieur est délimité par une paroi inférieure (11), une paroi de périmètre (12) et une paroi supérieure (13), lequel contenant comprend au moins une première bouche d'accès (21) ménagée sur ladite paroi supérieure (13) ou sur ladite paroi de périmètre (12), et des moyens (30, 40, 50) pour prélever l'urine recueillie dans le contenant, **caractérisé en ce que** lesdits moyens (30, 40, 50) pour prélever l'urine recueillie dans le contenant sont associés de manière permanente à ladite première bouche d'accès (21) et comprennent :
- un corps formant godet (30) inséré partiellement ou entièrement à l'intérieur dudit contenant (1) pour fermer ladite première bouche d'accès (21), ledit corps formant godet (30) délimitant une chambre (31) de réception d'un flacon sous vide (F), une ouverture (33) étant prévue sur le fond (32) dudit corps formant godet (30), qui met ladite chambre (31) en communication avec le volume intérieur (V) dudit contenant (1) ;
- des moyens (40) pour coupler pour la circulation de fluide ladite ouverture (33) avec ledit flacon (F) logé dans ladite chambre (31), lesdits moyens de couplage (40) étant associés au fond (32) dudit corps formant godet, dans ladite chambre (31), au niveau de ladite ouverture (33) ; et
- un tube (50) pour aspirer l'urine recueillie dans ledit contenant (1), lequel tube est couplé pour la circulation de fluide avec ladite ouverture (33) et s'étend à partir du fond (32) dudit corps formant godet (30) sensiblement jusqu'à ladite paroi inférieure (11) dudit contenant (1).

2. Contenant selon la revendication 1, dans lequel la distance (G) entre l'extrémité d'aspiration (51) dudit tube (50) et ladite paroi d'extrémité (11) est d'au moins 1 mm.

3. Contenant selon la revendication 1 ou 2, dans lequel ladite paroi inférieure (11) possède un renfoncement (14) au point où est placée l'extrémité d'aspiration (51) dudit tube (50), ledit renfoncement (14) facilitant la confluence des résidus d'urine présents dans ledit contenant (1) vers ladite extrémité d'aspiration (51).

4. Contenant selon la revendication 3, dans lequel ladite première bouche d'accès (21) est ménagée sur ladite paroi supérieure (13) sensiblement sur la perpendiculaire audit renfoncement (14).

5. Contenant selon l'une quelconque des revendications précédentes, dans lequel ledit tube (50) a un diamètre intérieur (Di) de 1 à 3 mm et une longueur (L) de 70 à 90 mm.

6. Contenant selon l'une quelconque des revendications 1 à 4, dans lequel ledit tube (50) a un diamètre intérieur (Di) de 1,2 à 3,5 mm et une longueur (L) de 140 à 180 mm.

7. Contenant selon l'une quelconque des revendications précédentes, dans lequel ledit corps formant godet (30) est retenu dans une position coaxiale dans ladite première bouche d'accès (21) au moyen d'une partie périphérique (35) qui est couplée au bord de ladite première bouche d'accès (21).

8. Contenant selon l'une quelconque des revendications précédentes, dans lequel lesdits moyens de couplage pour la circulation de fluide (40) comprennent une aiguille creuse sur sa longueur (41) qui dépasse dans ladite chambre (31) par le fond (32) dudit corps formant godet, ladite aiguille (41) étant couplée à sa base (41') pour la circulation de fluide avec ladite ouverture (33) et étant munie d'une pointe (41") pour perforer la membrane (M) fermant ledit flacon (F) afin de mettre l'intérieur dudit flacon (F) et le volume intérieur (V) dudit contenant (1) en communication pour la circulation de fluide au moyen de ladite ouverture (33).

9. Contenant selon la revendication 8, dans lequel lesdits moyens de couplage pour la circulation de fluide (40) comprennent une gaine (42) qui protège au moins la pointe (41") de ladite aiguille (41) et sert de barrière empêchant la fuite d'urine hors dudit contenant par ladite aiguille (41).

10. Contenant selon la revendication 8 ou 9, dans lequel ladite aiguille creuse sur sa longueur (41) a un diamètre intérieur (Di') de 0,75 mm à 1,6 mm.

11. Contenant selon la revendication 8, 9 ou 10, dans lequel ladite aiguille creuse sur sa longueur (41) a une longueur totale (L'tot) de 7 à 35 mm et une longueur de la partie dépassant à l'intérieur (L') de 2 à 30 mm.

12. Contenant selon une ou plusieurs des revendications 8 à 11, comprenant un pavillon (60) associé audit corps formant godet (30) sur ladite ouverture (33), lequel pavillon (60) associe ladite aiguille (41) et ledit tube (50) audit corps formant godet (30) en les reliant l'un à l'autre pour la circulation de fluide.

13. Contenant selon la revendication 12, dans lequel ledit pavillon (60) s'étend à l'intérieur de ladite chambre (31) avec une première partie (61) sur laquelle une première assise longitudinale (63) est formée pour insérer la base (41') de ladite aiguille (41), et à l'intérieur dudit contenant (1) avec une deuxième partie (62), sur laquelle une deuxième assise longitudinale (64) est formée pour insérer une première extrémité (50') dudit tube (50), lesdites deux assises d'insertion (63, 64) étant reliées l'une à l'autre pour la circulation de fluide par un canal longitudinal interne (65).

14. Contenant selon la revendication 13, dans lequel ledit pavillon (60) est muni d'une partie d'attache intermédiaire (66) entre ladite première partie (61) et ladite deuxième partie (62), au moyen de laquelle il est raccordé au fond (32) dudit corps formant godet (30) en correspondance avec une assise de fixation (34) qui est formée sur le fond (32) dudit corps formant godet (30) et qui définit ladite ouverture (33) dans le sens axial.

15. Contenant selon une ou plusieurs des revendications 12 à 14, dans lequel ledit pavillon (60) est muni d'un relief annulaire (68) qui est engagé à force dans un épaulement annulaire (37) de forme complémentaire formé dans ladite assise de fixation (34), en créant ainsi une barrière à la pénétration d'urine dudit contenant (1) dans ladite chambre de réception (31).

16. Contenant selon les revendications 14 et 15, dans lequel ledit relief annulaire (68) est formé à la base de ladite partie intermédiaire (66) et ledit épaulement annulaire (78) de forme complémentaire est formé à l'entrée de ladite assise de fixation (34).

17. Contenant selon l'une quelconque des revendications précédentes, comprenant une deuxième bouche d'accès (22).

18. Contenant selon l'une quelconque des revendications précédentes, comprenant une poignée de transport (70).

19. Contenant selon l'une quelconque des revendications précédentes, ayant un volume intérieur utile de 2 à 3,5 litres.
